# EUROPÄISCHE PATENTSCHRIFT

(11) **EP 0 224 890 B1**
(45) Veröffentlichungstag und Bekanntmachung des Hinweises auf die Patenterteilung: **13.10.1993**
(21) Anmeldenummer: 86116583.5
(22) Anmeldetag: 28.11.1986
(51) Int. Cl.: A61L 27/00, A61F 2/30, A61K 6/04, A61K 6/06, A61C 8/00

(54) **Aktives Implantat**
Active implant
Implant actif

(30) Priorität: 05.12.1985 DD 283775
(43) Veröffentlichungstag der Anmeldung: 10.06.1987
(73) Patentinhaber: AHC-Oberflächentechnik Friebe & Reininghaus GmbH, D-50171 Kerpen (DE)
(72) Erfinder: Kurze, Peter, Doz.Dr.sc.nat., DDR-9109 Oberlichtenau (DD); Krysmann, Waldemar, Dr.rer.nat., DDR-9071 Karl-Marx-Stadt (DD); Knöfler, Wolfram, Dr.-med., DDR-7030 Leipzig (DD); Graf, Hans-Ludwig, Dr.med., DDR-7024 Leipzig (DD); Bethmann, Wolfgang, OMR Prof.Dr.sc.med., DDR-4500 Dessau (DD); Hampel, Horst, Dr.med.dent., DDR-7010 Leipzig (DD)
(74) Vertreter: Zipse + Habersack

(56) Entgegenhaltungen:
- EP-A- 0 071 242
- DE-A- 2 324 867
- DE-A- 3 412 915
- FR-A- 2 336 913
- FR-A- 2 374 019
- US-A- 4 038 703

## Beschreibung

Die Erfindung betrifft ein aktives Implantat gemäß dem Oberbegriff des Anspruchs 1, das in der Medizin, insbesondere als Zahnimplantat oder Endoprothese, als auch in der Veterinärmedizin Verwendung findet.

Bei der Entwicklung von Implantaten, insbesondere Dentalimplantaten, hat sich herausgestellt, daß poröse Implantate besser vom Knochen eingebaut werden als glatte und ebene Formen (Mühlemann / Schweiz. Mschr. Zahnheilk. 85 (1975) 97 - 112/, Hodosh et al./J. Prothet. Dent. 24 (1970) 453 - 460/, Peterson er al./J. Dent. Res. 59 (1980) 99 - 10 -/). Es ist bekannt, daß dieses Prinzip durch Sintern gepreßter Drähte verfolgt wird (US-PS 3.906.550). Kontrollierte Porositäten werden auch durch Aufsintern von Drahtspiralen auf Substrate geschaffen (US-PS 4.038.703). Oberflächenrauhigkeiten werden durch Titanspritzschichten (DE-OS 26 28 485) und durch poröse Keramik geschaffen (DE-OS 27 54 917, DE-OS 27 33 394).

Der Nachteil der Sintermethode mit Drahtspiralen und gepreßten Drähten ist die nahezu völlige Unterbindung der Biegeverformung des Materials.
Weiterhin ist bekannt, daß Schichten von Kohlenstoff, Polymeren und Keramiken die Bioeigenschaften von Metallen verbessern, indem sie der Korrosion entgegenwirken und/oder bioaktive Vorgänge provozieren. Dem gleichen Zweck dienen Schichten aus Glimmpolymeren (DD-WP 156 462). Phthalozyaninen (DD-WP 156 312) und anderen Polymeren (DD-WP 133 518).

Der Nachteil dieses Implantates ist einerseits die teilweise geringe Haftfähigkeit der Schicht und andererseits die Unterbindug elektrischer Aktivitäten der Implantatmaterialien. Zudem benötigen alle Verfahren einen hohen technischen Aufwand. Es ist weiterhin bekannt, daß durch fließenden Strom das Knochenwachstum stimuliert werden kann (Bassett, Pawluk und Becker / Nature 204 (1964) 652 - 654 /, Cieszynski / Arch. Immunol. Exp. Thei. 11 (1963) 199 - 215 /).

Metall-Metalloxidübergänge bestimmter Metalle, z. B. Titan, generieren bei mechanischer Verformung ebenfalls elektrische Potentiale, die aber in Reizstromimplantaten nicht ausgenutzt und angewendet werden (Huber / Applied Physics letters 2 (1963) 76 - 78 /).

Aus der EP-A-0 071 242 ist eine künstliche Prothese bekannt, die einen Schaft aus Titan oder einer Legierung davon sowie ein diesen Schaft ummantelndes Titannetz enthält. Das Titannetz ist zum Korrosionsschutz mit Titanoxid überzogen. Die Prothese ist weiterhin mit einem biologisch verträglichen und abbaubaren, schwach elastischen Material, z.B. Kalziumphosphat, überzogen.

Die FR-A-23 36 913 beschreibt Knochen- und Zahnwurzelimplantate, die aus einem metallischen Basismaterial bestehen, das mit einer Hydroxylapatitschicht oder einer Mischung aus Hydroxylapatit und keramischem Material überzogen ist.

Aus der DE-A-23 24 867 ist die Aufgabenstellung bekannt, die Gewebefreundlichkeit einer Endoprothese zu verbessern. Diese Aufgabe wird in dieser Druckschrift dadurch gelöst, daß in die Implantatoberfläche Ionen eingebracht werden, die die Bildung einer mechanisch festen Verbindung vom Knochengewebe zum Implantat verbessern, stimulieren und beschleunigen sollen.

Es ist Aufgabe der Erfindung, Implantate herzustellen, die die Wachstumsmechanismen des Knochens positiv beeinflussen, eine lange Liegedauer erreichen und eine hohe biologische Akzeptanz aufweisen.

Diese Aufgabe wird bei einem Implantat der eingangs genannten Art erfindungsgemäß durch die kennzeichnenden Merkmale des Anspruchs 1 gelöst. Vorteilhafte Weiterbildungen der Erfindung sind Gegenstand der Unteransprüche.

Erfindungsgemäß wird ein aktives Implantat, bestehend aus biegsamen Metall- und/oder Keramikstrukturen, Draht- oder Foliengebilden, mit einem Oxid versehen, das als Wachstumsstimulatoren fungierende piezo-elektrisch wirksame Substanzen, beispielsweise Dihydrogenphosphate, Zirkonate, Tartrate, Titanate, Turmaline, Quarze u.a. enthält und das einen stabilen p-n-Übergang ermöglicht. Das Implantat kann auch aus einem derartigen Oxid bestehen.

Durch Kombination der materialimmanenten Elastizität von Metallen, Metallegierungen und Keramiken und dem piezoelektrischen Verhalten bestimmter Metall-Metalloxidübergänge oder Verbindungen, vorzugsweise der Elemente Al, Ti, Ta, Zr, Zb, Hf u.a., werden Deformationspotentiale ausgebildet. Die erfindungsgemäße Lösung der Ausbildung aktiver Implantate, insbesondere biegsamer Metall- und/oder Keramikstrukturen besteht darin, daß oberflächenstrukturierte, insbesondere grazile Zahnimplantate oder massive Endoprothesen mit dünnem T- und/oder Ta- und/oder Nb- und/oder Al- und/oder Zr- und/oder Hf-Draht und/oder -Folie umwickelt werden, oder aus diesen bestehen und diese mit einem signifikanten Oxid versehen sind, das Wachstumsstimulatoren wie Kalium- und/oder Ammoniumdihydrogenphosphate und/oder Zirkonate und/oder Turmaline und/oder Quarze und/oder Titane usw. enthält. Die gleichmäßige Oxidsahichtausbildung und Schaffung eines stabilen p-n-Überganges auf den flexiblen Strukturen, Draht- oder Foliegebilden oder auf Teilen dieser bei gleichzeitiger Dotierung des Oxides mit Wachstumsstimulatoren erfolgt erfindungsgemäß insbesondere mit Hilfe des Verfahrens der anodischen Oxidation unter Funkenentladung. Mit Hilfe dieses Schichtbildungsverfahrens werden auch die dünnen Drähte oder Folien auf dem Implantatgrundkörper aufgesintert und lokal fixiert.

Die flexiblen Strukturen mit ihren stabilen p-n-Übergängen sind derart dimensioniert, daß die jeweils auftretenden, vom Implantatort abhängigen, Kräfte zu einer reversiblen Verformung dieser Strukturen fuhren, Deformationspotentiale ausgebildet und durch den auftretenden Reizstromfluß das Knochen- und Gewebewachstum dauerhaft stimuliert werden.

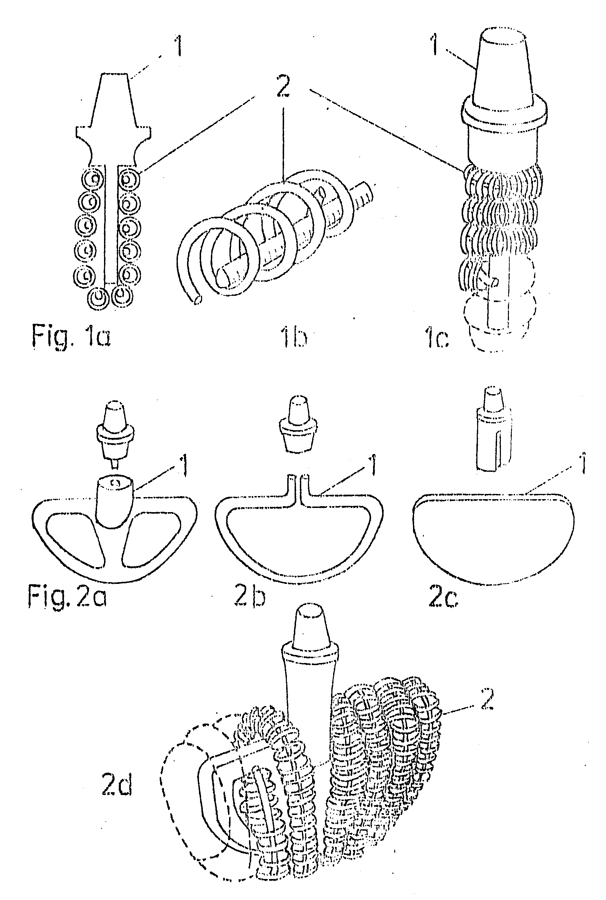

Die Erfindung soll nachstehend an Hand eines Ausführungsbeispieles näher erläutert werden. Es zeigen
- Fig. 1:: ein Einzelzahnimplantat a) Längsschnitt, b) biegsame spiralige Drahtkonstruktion, c) perspektivische Darstellung und
- Fig. 2:: ein Extensionsimplantat a) Grundkörper gegossen, b) Grundkörper als Drahtkonstruktion, c) Grundkörper aus Blech, d) perspektivische Darstellung des Extensionsimplantates.

Auf dem Grundkörper 1 aus Titan wird als flexible Struktur 2 eine Titan-Drahtspirale aufgebracht. Danach wird dieses Implantat zum Aufbringen des Oxides einer anodischen Oxidation unter Funkenentladung in einem Elektrolyten, welcher 0,6 molar an Fluorid, 0,5 molar an Dihydrogenphosphat, 0,1 molar an Tetraborat, 0,1 molar an Ammonium und 1,2 molar an Natrium ist und 10 g Bariumtitanat pro Liter Lösung enthält, bei 130 V und 1 A unterzogen. Dabei sintert die oxidierte Titan-Drahtspirale auf dem oxidierten Grundkörper 1 fest auf; die dabei entstehende, signifikant aufgerauhte Oxidschicht enthält Wachstumsstimulatoren des Elektrolyten. Die so ummantelte Titan-Drahtspirale zeigt bei Be- und Entlastung piezoelektrisches Verhalten, d. h. es bildet sich ein Deformationspotential aus, das einen Reizstromfluß zur Folge hat. Versuche zeigen, daß die Knochenbildung besonders um die Drahtspirale gut ausgebildet ist, was auf den Reizstrom zurückzuführen ist, der durch das langzeitfunktionelle Implantat garantiert wird. Die Intensität des Knochenkontaktes liegt um ca. 15 % höher als bei unbehandeltem Titan.

## Patentansprüche

1. Aktives Implantat, umfassend einen Grundkörper, der mit flexiblen Teilen aus Metall und/oder Keramik versehen ist oder daraus besteht,
dadurch **gekennzeichnet,**
daß die flexiblen Teile mit mit einem Oxid beschichtet sind bzw. daraus bestehen, das die Entstehung stabiler p-n-Übergänge ermöglicht und piezoelektrisch wirksame Substanzen enthält.

2. Aktives Implantat nach Anspruch 1,
dadurch **gekennzeichnet,**
daß die piezoelektrisch wirksamen Substanzen Dihydrogenphosphate, Zirkonate, Tartrate, Titanate, Turmaline und/oder Quarz enthalten.

3. Aktives Implantat nach Anspruch 1 oder 2,
dadurch **gekennzeichnet,**
daß das Oxid ein Oxid von Al, Ti, Ta, Zr, Nb oder Hf ist.

4. Aktives Implantat nach einem der vorherigen Ansprüche,
dadurch **gekennzeichnet,**
daß die flexiblen Teile aus Draht oder Folie gebildet sind.

5. Aktives Implantat nach einem der vorherigen Ansprüche,
dadurch **gekennzeichnet,**
daß die flexiblen Teile auf den Grundkörper aufgesintert sind.

6. Aktives Implantat nach einem der vorherigen Ansprüche,
dadurch **gekennzeichnet,**
daß das Oxid durch anodische Oxidation unter Funkenentladung gebildet ist.

## Claims

1. Active implant comprising a main body which is provided with or consists of flexible parts made of metal and/or ceramics,
**characterized** in
that said flexible parts are coated with or consist of an oxide which enables the generation of stable p-n-transitions and which contains piezoelectric materials.

2. Active implant according to claim 1,
**characterized** in
that the piezoelectric materials comprise dihydrogenphosphate, zirconates, tartrates, titanates, tourmalines and/or quartz.

3. Active implant according to claim 1 or 2,
**characterized** in
that the oxide is selected from the group Al, Ti, Ta, Zr, Nb, or Hf.

4. Active implant according to one of the precedent claims,
**characterized** in
that the flexible parts are formed of wire or foil.

5. Active implant according to one of the precedent claims,
**characterized** in
that the flexible parts are sintered on the main body.

6. Active implant according to one of the precedent claims,
**characterized** in
that the oxide is formed by use of anodic oxidation under spark discharge.

## Revendications

1. Implant actif comprenant un corps de base qui est muni ou qui se compose de parties flexibles en métal ou en céramique, caractérisé en ce que les parties flexibles sont enduites ou se composent d'un oxyde qui permet la formation de solides jonctions p-n et qui contient des substances actives du point de vue piézoélectrique.

2. Implant actif selon la revendication 1, caractérisé en ce que les substances actives du point de vue piézoélectrique contiennent des dihyrogénophosphates, des zirconates, des tartrates, des titanates, de la tourmaline et/ou du quartz.

3. Implant actif selon la revendication 1 ou 2, caractérisé en ce que l'oxyde est un oxyde de Al, Ti, Ta, Zr, Nb, ou Hf.

4. Implant actif selon l'une des revendications précédentes, caractérisé en ce que les parties flexibles sont en fil ou en feuille.

5. Implant actif selon l'une des revendications précédentes, caractérisé en ce que les parties flexibles sont appliquées par frittage sur le corps de base.

6. Implant actif selon l'une des revendications précédentes, caractérisé en ce que l'oxyde est formé par oxydation anodique avec décharge par étincelles.
